# EUROPEAN PATENT SPECIFICATION

(11) **EP 2 601 951 B1**
(45) Date of publication and mention of the grant of the patent: **29.04.2015**
(21) Application number: 10855546.7
(22) Date of filing: 10.12.2010
(51) Int. Cl.: A61K 31/4415, A61K 31/198, A61K 31/405, A61K 31/4172, A61K 31/401, A61K 31/197, A61K 31/375, A61K 31/51, A61K 31/525, A61K 31/455, A61K 31/4188, A61K 31/519, A61K 31/714, A61P 7/04

(54) **PHARMACEUTICAL COMPOSITION FOR TREATING HEMORRHAGE CAUSED BY BLOOD CLOTTING DISORDER AND USE THEREOF**
PHARMAZEUTISCHE ZUSAMMENSETZUNG ZUR BEHANDLUNG VON DURCH BLUTGERINNUNGSUNREGELMÄSSIGKEITEN VERURSACHTEN HÄMORRHAGEN
COMPOSITION PHARMACEUTIQUE DESTINÉE AU TRAITEMENT D'UNE HÉMORRAGIE PROVOQUÉE PAR UN TROUBLE DE LA COAGULATION ET UTILISATION ASSOCIÉE

(30) Priority: 06.08.2010 CN 201010248451
(43) Date of publication of application: 12.06.2013
(73) Proprietor: Yue, Maoxing, Chaoyang, Beijing 100101 (CN); Wan, Honggui, Jiangsu 210009 (CN); Huang, Tongge, Jiangsu 210000 (CN)
(72) Inventor: Yue, Maoxing, Chaoyang, Beijing 100101 (CN); Wan, Honggui, Jiangsu 210009 (CN); Huang, Tongge, Jiangsu 210000 (CN)
(74) Representative: Kramer Barske Schmidtchen Patentanwälte PartG mbB
(86) International application number: PCT/CN2010/079678
(87) International publication number: WO 2012/016408

(56) References cited:
- CN-A- 1 640 396
- US-A- 4 657 866
- US-A1- 2007 286 909
- US-A1- 2009 076 111

## Description

### Technical Field

The present invention belongs to the emergency treatment field of critically ill patients and involves a pharmaceutical composition useful for treating coagulation disorder hemorrhage for patients bleeding due to coagulation disorder.

### Background Technology

In the case of severe trauma and infection, the blood system is often affected. The coagulation system abnormalities and dysfunction are very common in the clinical manifestations of systemic inflammatory response syndrome (SIRS) and multiple organ dysfunction syndrome (MODS). The blood system involvement in MODS ranks the top four in the organs involved in the literature reports and ranks the second in individual literature reports. The blood system abnormalities become the main cause and direct cause of the patients' death. The abnormal changes in the blood system during multiple organ dysfunction and failure are mainly divided into the changes in formed elements and the changes in coagulation and hemostatic functions. The coagulation dysfunction can be the inevitable result of disease occurrence and development and can also be induced by inappropriate treatment. In the case of massive hemorrhage caused by coagulation dysfunction in MODS, doctors are often helpless and most patients will die soon. Therefore, it is very important to research the key technologies for rescuing such dying patients.

Under normal circumstances, the coagulation process of organism is usually divided into intrinsic pathway, extrinsic pathway and common pathway.

The intrinsic coagulation pathway means that all the participatory coagulation factors come from the blood (intrinsic). When the vessel wall is injured, the subendothelial tissue is exposed and the negatively charged subendothelial collagenous fibers are in contact with the coagulation factors, the factor XII will be combined with the coagulation factors and be activated as XIIa under the participation of HK and PK. The factor XIIa will activate the factor XI without Ca²⁺ and the activated XIa will activate the factor IX with the presence of Ca²⁺. With fairly low effectiveness, the separate IXa activating factor X shall be combined with VIIIa to form the 1:1 complex, also known as the factor X enzyme complex. Ca²⁺ and PL must participate in this reaction jointly. The intrinsic coagulation pathway, in fact, refers to the process from the factor XII is activated to the factor X is activated.

The extrinsic coagulation pathway means that not all the participatory coagulation factors exist in the blood and there are extrinsic coagulation factors participating in hemostasis. In this process, the tissue factor is initiated due to exposure to the blood, such that the factor X is activated. As a specific transmembrane protein existing in a variety of plasma membranes, the tissue factors is released after the tissue is damaged and forms the 1:1 complex with the factor VII in the participation of Ca²⁺. The factor VII combined with the tissue factor will be soon activated as VIIa by the activated factor X. The extrinsic coagulation pathway is mainly adjusted by the tissue factor pathway inhibitor (TFPI), which is a kind of glycoprotein existing in normal human plasma and blood platelets and vascular endothelial cell and forms the complex in combination with the factor Xa or factor VIIa-tissue factor-factor Xa to inhibit the activity of the factor Xa or factor VIIa-tissue factor. In addition, studies have shown that the intrinsic coagulation and extrinsic coagulation pathways can be activated mutually.

From the activation of the factor X to the formation of fibrin is the common coagulation pathway of intrinsic and extrinsic coagulation, mainly including two stages of thrombin generation and fibrin formation.
(1) Thrombin generation: the factor Xa and factor Va form the thrombinogen complex, that is, the thrombokinase in the presence of Ca²⁺ and phospholipid membranes and transform the thrombinogen into thrombin.
(2) Fibrin formation: the fibrinogen is decomposed into fibrin monomers by the thrombin by means of enzymolysis and forms stable fibrin clot by cross-linking. This process can be divided into three stages, fibrin monomer generation, fibrin monomer polymerization and fibrin monomer cross-linking. The negatively charged fibrinopeptide A and fibrinopeptide B are transformed into fibrin monomers after hydrolyzed by the thrombin. After generation, the fibrin monomers are combined in non-covalent bonds to form the fibrin polymer, also known as soluble fibrin. After generation, the fibrin can promote the activation of the thrombin to the factor XIII. With the involvement of XIIIa and Ca²⁺, the adjacent fibrins are cross-linked rapidly to form the insoluble stable fibrin clots.

Since the MODS pathogenesis has not been fully elucidated and the clinical treatment has not made a breakthrough, the fatality rate of MODS remains high and especially the coma, emergency ulcer massive hemorrhage, etc. resulted from MODS cause that the traditional coagulation drugs can not play the role directly. Therefore, the organs with high costs are generally used to support the extracorporeal circulation adjuvant therapy.

It is reported from literature that four major coagulation factors (factor II, factor V, factor VII and factor VIII) in the coagulation system are synthesized by the liver. Under MODS, the hyperbilirubinemia, aminotransferases (ALT or AST) and LDH rise and the prothrombin time is prolonged with jaundice and flapping tremor; the platelet count is less than 80000/ul or falls by over 50% in three days; the blood pressure of arteria digitalis is no more than or the mean arterial pressure is no more than 70mmHg, accompanied with tachycardia, arrhythmia, cardiac arrest and so on. The existence of a variety of harmful free radicals aggravates the damages to the organs and especially brings series damage to the liver, resulting in increased blood ammonia concentration, slow metabolism, blocked synthesis of key enzymes and important factors (4 major coagulation factors) and bringing life threat to the patients in massive hemorrhage under MODS.

US 4,657,866 A discloses serum-free, synthetic, completely chemically defined tissue culture media.

US 2007/0286909 A1 relates to a nutritional composition comprising a homogenous mixture of free amino acids, wherein the free-form amino acids comprise L-Lysine, L-Valine, L-Tryptophan or a metabolite thereof, L-Phenylalanine, L-Methionine, L-Leucine, L-Threonine, L-Isoleucine, L-Arginine, L-Histidine, L-Tyrosine, L-Carnitine, L-Serine, L-Glutamine, Aspartic Acid, L-Proline, L-Glycine, Taurine, L-Cysteine, Gamma-aminobutyric acid (GABA), L-Alanine, L-Glutamic acid, and wherein the composition comprises at least one B vitamin.

US 2009/0076111 A1 relates to a method for improving glycemic control in mammals, comprising the step of administering an amount of a composition comprising an amino acid content including 4-hydroxyisoleucine and glutamate, wherein said 4-hydroxyisoleucine comprises an amount between about 60 % and about 70 % of a total weight of said amino acid content and said cysteine comprises between about 1 % and about 2 % of the total weight of the amino acid content.

### Invention Content

The purpose of the present invention is to provide a pharmaceutical composition useful for treating hemorrhage caused by coagulation disorder, and the application thereof in the preparation of drugs used to treat coagulation disorder hemorrhage.

The action mechanism of the present invention: as the protective agent of brain and nerve and also a natural diuretic, the large doses of vitamin B6 need to participate in more than 60 kinds of known enzymes. With vitamin C, the vitamin B can remove the harmful free radicals generated by MODS and quickly reduce the poison of free oxygen to the organs. The compound amino acid containing L-ornithine, aspartic acid and arginine can provide the appropriate substrate for the intrinsic coagulation mechanism. With the included high branched chain amino acid, it can not only correct the metabolic imbalance between the branched chain amino acid and aromatic amino acid, but also inhibit the formation of false neurotransmitters in the brain and improve hepatic encephalopathy. L-ornithine itself can quickly penetrate the mitochondrial membrane, carry a molecule of carbon dioxide and a molecule of ammonia to transform into L-citrulline through metabolism in the mitochondria, rapidly activate the urea cycle in liver cells with L-aspartic acid after rapidly passing through the mitochondria and discharge the harmful carbon dioxide and ammonia generated by the body outside under MODS by ornithine metabolism (urea cycle), resulting in the gradual recovery of enzyme metabolism in the liver, the generation of four major coagulation factors and rapid recovery of intrinsic coagulation pathway. This method with conventional treatment can effectively treat the dying patients in massive hemorrhage due to coagulation disorders.

The purposes of the present invention are achieved through the following technical schemes:
A pharmaceutical composition useful for treating coagulation disorder hemorrhage, containing L-ornithine 0.5~8g, aspartic acid 1~5g, arginine 3~10g and vitamin B6 3-10g per unit.
Said pharmaceutical composition may also contain one or more of the following substances per unit: isoleucine, leucine, lysine, methionine, phenylalanine, threonine, tryptophan, valine, histidine, glycine, alanine, proline, asparagine, cysteine, glutamic acid, serine, tyrosine, VitB1, VitB2, VitB3, pantothenic acid, biotin, folic acid, VitB12 and vitamin C;
wherein the dosages of amino acids are respectively: isoleucine 3~10g, leucine 5~15g, lysine 3~10g, methionine 0.5-3g, phenylalanine 0.5~3g, threonine 3~10g, tryptophan 0.5~3g, valine 5~15g, histidine 3~8g, glycine 3~8g, alanine 3~10g, proline 3~8g, asparagine 0.1~3g, cysteine 0.1~3g, glutamic acid 3~10g, serine 0.5~5g, tyrosine 0.1∼3g; the dosages of B vitamins are respectively: VitB1 1~2mg, VitB2 1~2mg, VitB3 10~20mg, pantothenic acid 3∼5mg, biotin 0.1~0.2mg, folic acid 0.1~0.4mg, VitB12 2~6µg; vitamin C1-3g.

Said pharmaceutical composition may also contain an appropriate amount of 5% sodium chloride and dextrose injection solution or 0.9% sodium chloride injection solution.

The application of the said pharmaceutical composition in the preparation of drugs used to treat coagulation disorder hemorrhage.

The above pharmaceutical composition is infused at the appropriate time.

Mild patients use 0.5~1 unit of the above pharmaceutical composition each day and are dosed with intravenous injection for 1-9 consecutive days with 30 minutes to 6 hours each day; the severe patients use 0.5~1 unit of the above pharmaceutical composition each day, are dosed with intravenous injection for 1-5 consecutive days with 20-30 minutes each day and use the treatment method of mild patients as above after symptoms improve; the extra severe patients use 1~2 units of the above pharmaceutical composition each day, are rapidly dosed in central vein for 1-5 consecutive days with 10-15 minutes each day and use the treatment method of mild patients as above after symptoms improve.

The said amino acids in the present invention are L-amino acids unless expressly stated.

### Beneficial effects of the present invention:

The present invention ingeniously uses the shock therapy with the compound amino acid injection containing L-ornithine, aspartic acid and arginine + a large dose of B vitamins combined with vitamin C as the patients' conditions may be, efficiently opening up the body's metabolic pathways under MODS state and providing a cost-effective method for saving the dying patients in coagulation disorder hemorrhage. This method has not been reported in the literature at home and abroad.

By using the shock therapy with the compound amino acid injection containing L-ornithine, aspartic acid and arginine + a large dose of B vitamins to treat hundreds of emergency and critical disease patients, the total protein (TP), albumin (ALB), total bilirubin (TBIL), indirect bilirubin (IBIL), alanine transaminase (ALT) and aspartate aminotransferase (AST) have declined significantly, the coagulation function and HB have risen, the immunologic function has been improved significantly and the globulin (GLB), white/ball (A/B) and direct bilirubin (DBIL) have not changed significantly. It can be seen that the new therapy with the compound amino acid injection containing L-ornithine, aspartic acid and arginine combined with a large dose of B vitamins can really play an important role in the treatment of critical diseases and is quite helpful to the recovery of liver function, increase of coagulation function and HB and improvement of immunological function.

Creatively use the shock therapy of the compound amino acid injection containing L-ornithine, aspartic acid and arginine combined with a large dose of B vitamins for treatment on the basis of conventional treatment:
1) For the hemorrhagic patients with the pathogens causing MODS and caused by coagulation dysfunction due to infectious and non-infectious factors (severe trauma, severe acute pancreatitis, major surgery, cardiopulmonary resuscitation, pathological pregnancy, etc.)
2) For the hemorrhagic patients caused by concurrent coagulation dysfunction of abnormal liver function
3) For the hemorrhagic patients caused by concurrent coagulation dysfunction of other diseases. It also has secondary efficacy for the organism's metabolic disturbance caused by severe trauma, especially the multiple organ failure under MODS state.

### Specific Execution Mode

Next, further elaboration of the present invention is presented through embodiments.

### Embodiment 1: Statistics of clinical treatment

### I. Case inclusion criteria:

(1) For the hemorrhagic patients with the pathogens causing MODS and caused by coagulation dysfunction due to infectious and non-infectious factors (severe trauma, severe acute pancreatitis, major surgery, cardiopulmonary resuscitation, pathological pregnancy, etc.)
(2) The system and organ disorder occurs when the integral of single organ damage is no less than 1 and the system and organ failure occurs when the integral of single organ damage is no less than 3 according to Marshall Standard (Table 1) 24 hours after the occurrence of above factors.
(3) Age between 18 and 86.
(4) For the hemorrhagic patients caused by the coagulation dysfunction due to MODS and liver dysfunction.

In the present invention, the judgment standard for the mild patients, severe patients and extra severe patients is the marking criteria development by Canadian Marshall professor in 1995. The higher the score, the more serious the illness condition, as shown in Tables 1 and 2.

**Table 1 MODS Marking Criteria (Marshall 1995)**

| | 0 | 1 | 2 | 3 | 4 |
|---|---|---|---|---|---|
| Respiratory system (PaO₂/FIO₂) | >300 | 226-300 | 151-225 | 76-150 | ≤75 |
| Kidney (Serum creatinine µmol/L) | ≤100 | 101-200 | 201-350 | 351-500 | >500 |
| Liver (Hemobilirubin mg/L) | ≤20 | 21-60 | 61-120 | 121-240 | >240 |
| Cardiovascular (PAR) | ≤10.0 | 10.5-15.0 | 15.1-20.0 | 20.1-30.0 | ≥30.0 |
| Blood (Blood plateletx 10⁹) | >120 | 80-120 | 51-80 | 21-50 | ≤ 20 |
| Central nervous system (Glasgow Marking) | 15 | 13-14 | 10-12 | 7-9 | ≤6 |

| | | | | | |
|---|---|---|---|---|---|
| Note: PAR (heart rate after pressure adjustment) = heart rate x right atrial (central vein) pressure/ mean blood pressure | | | | | |

| Table 2 Judgment of Hemorrhagic Shock Degree and Estimated Blood Loss | | | |
|---|---|---|---|
| Shock degree* | Mild (compensatory stage) | Severe patients | Extra severe patients |
| Consciousness | Lucid | Faint | Blurred |
| Skin color, temperature | | Pale, cold | Gray, wet cold |
| Superficial vein | | Shrinkage, thinning | Collapse |
| Pulse (beats/min) | Faster | 100∼120 | > 120, thin and delicate |
| Systolic blood pressure | Normal | 70~90, narrow pulse pressure | < 70, narrower pulse pressure |
| Urine volume | | Oliguria | <25ml/h |
| Estimated blood loss (ml)** | 600~800 | 800∼1600 | > 1600 |
| Ratio of hemorrhage volume to total blood volume (%) | 20 | 35 | >40 |
| *The mild shock is in the shock compensatory stage and the medium and severe shock is in the shock decompensatory stage. | | | |
| **Calculated by the blood volume of 7% of the weight, such as a wounded person of 50kg with the blood volume of 4000ml, the hemorrhage volume of 700ml is 20% of the weight. | | | |

Combined with Tables 1 and 2, the mild patients are with the MODS marking of level 0∼1, the severe patients are with the MODS marking of level 2∼3 and the extra severe patients are with the MODS marking of level 4. The illness conditions of the patients are distinguishes as mild, severe and extra severe according to the representation of hemorrhagic shock.

### II. Exclusion criteria:

(1) For the patients dead with the treatment of no more than 24 hours or discharges;
(2) For the patients failing to adhere to systemic treatment.

### III. Judgment criteria for coagulation function improvement:

(1) Extract 15ml venous blood after treatment for 0, 1, 4, 7, 14 and 28 days, centrifuge at the speed of 3000 rev/min, separate the serum and store in a low-temperature refrigerator for uniform testing.
(2) Detect PLT, D-Dimer, FDP, Fib, whole blood coagulation time (CT).
(3) Observe the time when actual hemorrhage stops.
(4) Liver: ALT, AST, PT and prothrombin activity, TBIL, albumin and cholinesterase.

### IV. Judgment criteria for curative effect:

1. Ineffective: there is no effect or the coagulation function is only slightly improved and the hemorrhage symptom can not be relieved in combination with conventional treatment;
2. Effective: the coagulation function of the mild patients is significantly improved in combination with conventional treatment and the hemorrhage symptom is relieved within 1-9 days;
3. Tangibly effective: the coagulation function of the severe and extra severe patients significantly improved in combination with conventional treatment and the hemorrhage symptom stops within 1-5 days.

The conventional treatment in the present invention refers to the use of haemostatic drugs for hemorrhagic patients, including platelets, prothrombin complexes, cryoprecipitation, plasma and a series of haemostatic methods. The aorta rupture hemorrhage has been stanched through surgeries.

### V. Treatment methods:

Per unit the pharmaceutical composition contains: L-ornithine 0.5~8g, aspartic acid 1~5g, arginine 3~10g and vitamin B6 3-10g;

Per unit the pharmaceutical composition also contains one or more of the following substances: isoleucine 3~10g, leucine 5~15g, lysine 3~10g, methionine 0.5~3g, phenylalanine 0.5~3g, threonine 3~10g, tryptophan 0.5~3g, valine 5~15g, histidine 3~8g, glycine 3~8g, alanine 3~10g, proline 3~8g, asparagine 0.1~3g, cysteine 0.1~3g, glutamic acid 3~10g, serine 0.5~5g, tyrosine 0.1~3g; the dosages of B vitamins are respectively: VitB1 1~2mg, VitB2 1~2mg, VitB3 10~20mg, pantothenic acid 3~5mg, biotin 0.1~0.2mg, folic acid 0.1~0.4mg, VitB12 2∼6µg; vitamin C1-3g.

The above substances can be added in 250ml∼500ml of 5% sodium chloride and dextrose injection solution or 0.9% sodium chloride injection solution (0.9% sodium chloride injection solution for diabetic patients).

Combined with conventional treatment, the mild patients use 1 unit of the above pharmaceutical composition each day and are dosed with intravenous injection for 1-9 consecutive days with 30 minutes to 6 hours each day;

Combined with conventional treatment, the severe patients use 0.5∼1 unit of the above pharmaceutical composition each day, are dosed with intravenous injection for 1-5 consecutive days with 20-30 minutes each day and use the treatment method of mild patients as above after symptoms improve;

Combined with conventional treatment, the extra severe patients use 1∼2 units of the above pharmaceutical composition each day, are rapidly dosed in central vein for 1-5 consecutive days with 10-15 minutes each day and use the treatment method of mild patients as above after symptoms improve.

### VI. Treatment effect:

Select 156 cases of mild patients, 68 cases of severe patients and 21 cases of extra severe patients with 169 male and 76 female, the age of 18∼86 and the average age of 57;
180 effective cases, 55 tangibly effective cases, 8 ineffective cases and 2 excluded cases.

### Embodiment 2: Presentation of specific cases

1. Mr. Zhu, 66 years old, with gallstones, had normal coagulation function before surgery. He was satisfied with the treatment of blood vessels and gallbladder bed in the cholecystectomy which was operated smoothly. Everything was normal in the morning on the day of the surgery and he vomited in the evening and then the blood appeared in the abdominal drain tube with faster heartbeat and decreased blood pressure. He was operated on again in that evening. It was found in the surgery that the vascular ligature did not fall off and only there were hemorrhagic spots in the gallbladder bed with peripheral edema, which was suspected to be caused by tear after vomit. The gallbladder bed was carefully stitched and rinsed repeatedly. The abdomen was closed and the surgery was completed after all medical personnel beside the operating table observed for half and hour and confirmed no hemorrhage. At that night and the next morning, the abdominal drainage amount was not much and normal, but in the evening the abdominal drainage amount was significantly increased and the hemorrhage was obvious, so the third surgery was conducted. There were no obvious hemorrhagic spots in the abdominal cavity after it was opened, and only the liver, the liver surrounding tissues and the retroperitoneum had obvious edema with slow hematopedesis. The hemostasis by compression, stitch hemostasis, electric coagulation hemostasis and other conventional means did not work. Thus, the patient was continuously dosed with a large number of hemostatic drugs, including platelets, prothrombin complexes, cryoprecipitation, plasma, etc. Then the hemorrhage was decreased slightly but continued. He was treated and cured by means of shock therapy of compound amino acid injection 500ml (including L-ornithine 2.80g, aspartic acid 2.50g, arginine 8.50g, isoleucine 7.80g, leucine 12.50g, lysine 7.50g, methionine 1.80g, phenylalanine 1.60g, threonine 4.60g, histidine 4.50g, glycine 5.50g, proline 6.60g, asparagine 1.20g, cysteine 0.80g, glutamic acid 5.90g, tyrosine 1.20g) and a large dose of vitamin B6 (250mL 5% sodium chloride and dextrose injection added with 50 pieces of vitamin B6 (5g) and 2 pieces of vitamin C(2g)) on the basis of conventional hemostatic measures and was given venous transfusion for 30 min. Half an hour later, the patent's liver and the surrounding edema had subsided and the abdominal hemorrhage had also been reduced. An hour later, the hemorrhage was less, so the abdomen was closed and the surgery was completed. The patient was successfully discharged after the surgery.
2. Mr. Fang, 41 years old, was subject to splenectomy and portal azygos disconnection surgeries four months ago due to cirrhosis, portal hypertension, hypersplenism, esophageal phleborrhexis massive hemorrhage. The massive hemorrhage appeared again after the surgery and the patient was discharged after active treatment in stable condition. The massive hemorrhage appeared again after a bath four days ago and the lowest hemachrome was 4.8g. The massive hemorrhage appeared again after the illness state was stable one. After blood transfusion, plasma transfusion, protein and hemostatic drugs, the patient's hemachrome was increased to 7.3g, but still with a large number of ascites, renal insufficiency and massive hemorrhage. The critical condition process of the patient was inhibited by means of immune nutritional support, microcirculation, and antagonism of inflammatory mediators, gradual recovery with metabolic enzymes and other treatment measures with the method of local and systemic treatment combination according to the illness condition. The patient used diuretic measures appropriately, used compound amino acid injection 500ml each day (including L-ornithine 1.85g, aspartic acid 2.50g, arginine 8.80g, isoleucine 8.80g, leucine 13.60g, lysine 7.51g, phenylalanine 1.60g, threonine 4.60g, tryptophan 1.50g, valine 10.60g, histidine 4.70g, glycine 6.30g, alanine 8.30g, proline 7.10g, asparagine 0.48g, glutamic acid 5.70g, serine 3.70g, tyrosine 0.67g) and vitamin B6 8g (added to 250mL 5% sodium chloride and dextrose injection), and was given venous transfusion for 4 consecutive days with 2 hours each day by means of shock therapy. The patient's condition had been stabilized and then the hemorrhage stopped and the patient was saved.
3. Ms. Yang, 85 years old with the patient number of 752, had extensive cerebral infarction, severe diabetes and serious pulmonary infection causing MODS and coma for five days. The critical condition process of the patient was inhibited by means of immune nutritional support, microcirculation, and antagonism of inflammatory mediators, gradual recovery with metabolic enzymes and other treatment measures. The patient used the compound amino acid injection 500mL each day (including L-ornithine 4.50g, aspartic acid 2.80g, arginine 8.50g, isoleucine 7.50g, leucine 10.80g, lysine 8.50g, methionine 1.60g, phenylalanine 2.00g, threonine 4.60g, tryptophan 1.50g, valine 10.50g, histidine 4.70g, glycine 6.30g, alanine 8.00g, proline 6.50g, asparagine 0.60g, cysteine 0.80g, glutamic acid 5.00g, serine 3.50g, tyrosine1.60g) + vitamin B6 10g + vitamin B1 1.5mg + vitamin B2 1.5mg + vitamin C 2g (vitamin was added to 250mL 0.9% sodium chloride injection), and was given venous transfusion for 2 consecutive days with 30 minutes each day by means of shock therapy in combination with a short-range large dose of anisodamine and dexamethasone (3 consecutive days with 0.66mg/kg/time respectively and 3 times/day). The conjunctive use of a large dose of anti-aerobic and anti-anaerobic antibiotics made Ms. Yang, an extra severe patient through the strike of MODS and thus come around. The patient was improved by continuous use of the above compound amino acid injection 500ml+vitamin B6 10g + vitamin C 2g for 3 consecutive days with 3 hours each day.
4. Mr. Lee, 84 years old, required long-term dialysis every two days due to chronic renal failure and had cerebral infarction, hypertension and severe diabetes. The patient was in a coma as the gastrointestinal tract appeared stress ulcer hemorrhage due to MODS caused by severe pulmonary infection. The key technology used to rescue the dying wounded was intended to use, that is, the patient used the compound amino acid injection 500mL each day (including L-ornithine 3.5g, aspartic acid 2.50g, arginine 8.80g, isoleucine 8.80g, leucine 13.60g, lysine 7.51g, methionine 1.20g, phenylalanine 1.60g, threonine 4.60g, tryptophan 1.50g, valine 10.60g, histidine 4.70g, glycine 6.30g, alanine 8.30g, proline 7.10g, asparagine 0.48g, cysteine 0.59g, glutamic acid 5.70g, serine 3.70g, tyrosine 0.67g) + vitamin B 6 5g + vitamin C 2g (vitamin was added to 250mL 0.9% sodium chloride injection) and was given venous transfusion for 3 consecutive days with 30 minutes each day by means of shock therapy with the method of local and systemic treatment combination. The successful method stopped the stress ulcer hemorrhage and the patient came around with stable vital signs. The patient was improved by continuous use of the above compound amino acid injection 500ml+vitamin B6 6g + vitamin C 2g for 5 consecutive days with 4 hours each day.

### Embodiment 3: Pharmaceutical composition preparation case

A pharmaceutical composition with the components of compound amino acid injection containing 5 kinds of amino acids (in which, L-ornithine content of 1.5g, L-aspartic acid content of 2.5g, L-arginine content of 8.5g, L-serine content of 3.8g and L-threonine content of 4.6g) and Vit B6 8g.

### Embodiment 4: Pharmaceutical composition preparation case

A pharmaceutical composition with the components of compound amino acid injection 500mL (including L- ornithine 3.5g, aspartic acid 2.50g, arginine 8.80g, isoleucine 8.80g, leucine 13.60g, lysine 7.51g, methionine 1.20g, phenylalanine 1.60g, threonine 4.60g, tryptophan 1.50g, valine 10.60g, histidine 4.70g, glycine 6.30g, alanine 8.30g, proline 7.10g, asparagine 0.48g, cysteine 0.59g, glutamic acid 5.70g, serine 3.70g, tyrosine 0.67g), Vit B6 3-10g, Vit C 1-3g and 0.9% sodium chloride injection 250mL with vitamin added.

### Embodiment 5: Pharmaceutical composition preparation case

A pharmaceutical composition with the components of compound amino acid injection 500mL (including L-ornithine 4.5g, aspartic acid 2.80g, arginine 8.30g, isoleucine 6.50g, leucine 12.00g, lysine 7.50g, methionine 1.60g, phenylalanine 1.40g, tryptophan 1.80g, valine 10.60g, histidine 4.80g, glycine 6.20g, alanine 8.50g, proline 7.10g, asparagine 0.48g, glutamic acid 5.70g, serine 3.70g, tyrosine 0.67g), Vit B6 3-10g, VitB1 1∼2mg, VitB2 1∼2mg, VitB3 10∼20mg, pantothenic acid 3∼5mg, biotin 0.1∼0.2mg, folic acid 0.1∼0.4mg, VitB12 2∼6µg; Vit C 1-3g and 5% sodium chloride and dextrose injection 250mL with vitamin added.

### Embodiment 6: Pharmaceutical composition preparation case

A pharmaceutical composition with the components of compound amino acid injection 500mL (including L-ornithine 2.5g, aspartic acid 2.50g, arginine 8.80g, isoleucine 6.80g, leucine 11.50g, lysine 7.50g, methionine 1.60g, phenylalanine 1.30g, threonine 4.40g, tryptophan 1.70g, histidine 4.60g, glycine 6.30g, alanine 8.30g, proline 6.20g, asparagine 0.60g, cysteine 0.80g, glutamic acid 5.70g, serine 3.70g, tyrosine 1.10g), VitB1 1∼2mg, VitB2 1∼2mg, Vit B6 3-10g, Vit C 1-3g, and 5% sodium chloride and dextrose injection 250mL with vitamin added.

## Claims

1. A pharmaceutical composition useful for treating coagulation disorder hemorrhage, the composition containing L-ornithine 0.5~8g, aspartic acid 1~5g, arginine 3∼10g and vitamin B6 3-10g per unit.

2. The pharmaceutical composition according to claim 1, also containing one or more of the following substances per unit: isoleucine, leucine, lysine, methionine, phenylalanine, threonine, tryptophan, valine, histidine, glycine, alanine, proline, asparagine, cysteine, glutamic acid, serine, tyrosine, VitB1, VitB2, VitB3, pantothenic acid, biotin, folic acid, VitB12 and vitamin C;
wherein the dosages of amino acids are respectively: isoleucine 3∼10g, leucine 5~15g, lysine 3~10g, methionine 0.5~3g, phenylalanine 0.5~3g, threonine 3∼10g, tryptophan 0.5~3g, valine 5~15g, histidine 3~8g, glycine 3~8g, alanine 3~10g, proline 3~8g, asparagine 0.1~3g, cysteine 0.1~3g, glutamic acid 3∼10g, serine 0.5~5g, tyrosine 0.1~3g; the dosages of B vitamins are respectively: VitB1 1~2mg, VitB2 1~2mg, VitB3 10~20mg, pantothenic acid 3~5mg, biotin 0.1~0.2mg, folic acid 0.1~0.4mg, VitB12 2~6µg; vitamin C 1-3g.

3. The pharmaceutical composition according to claim 1, also containing an appropriate amount of 5% sodium chloride and dextrose injection solution or 0.9% sodium chloride injection solution.

4. The application of the pharmaceutical composition according to claim 1, 2 or 3 in the preparation of drugs used to treat coagulation disorder hemorrhage.

## Patentansprüche

1. Die pharmazeutische Lösung zur Behandlung von Blutungen aufgrund von Gerinnungsstörungen enthält 0,5~8 g L-Ornithin, 1∼5 g Asparaginsäure, 3∼10 g Arginin und 3-10 g Vitamin B6 pro Einheit.

2. Gemäß Anspruch 1 enthält die pharmazeutische Lösung pro Einheit eine oder mehrere der folgenden Substanzen: Isoleucin, Leucin, Lysin, Methionin, Phenylalanin, Threonin, Tryptophan, Valin, Histidin, Glycin, Alanin, Prolin, Asparagin, Cystein, Glutaminsäure, Serin, Tyrosin, Vitamin B1, Vitamin B2, Vitamin B3, Pantothensäure, Biotin, Folsäure, Vitamin B12 und Vitamin C,
wobei die Dosis von Aminosäuren wie folgt verteilt ist: 3∼10 g Isoleucin, 5∼15 g Leucin, 3~10 g Lysin, 0,5~3 g Methionin, 0,5~3 g Phenylalanin, 3∼10 g Threonin, 0,5∼3 g Tryptophan, 5∼15 g Valin, 3-8 g Histidin, 3-8 g Glycin, 3∼10 g Alanin, 3-8 g Prolin, 0,1∼3 g Asparagin, 0,1∼3 g Cystein, 3∼10 g Glutaminsäure, 0,5~5 g Serin, 0,1∼3 g Tyrosin. Die Dosierung von B-Vitaminen ist wie folgt: 1-2 mg Vitamin B1, 1-2 mg Vitamin B2, 10∼20 mg Vitamin B3, 3-5 mg Pantothensäure, 0,1∼0,2 mg Biotin, 0,1∼0,4 mg Folsäure, 2-6 µg Vitamin B12, 1-3 g Vitamin C

3. Die pharmazeutische Lösung enthält gemäß Anspruch 1 eine entsprechende Menge an 5 % Natriumchlorid und einer Glucoseinjektionslösung bzw. 0,9 % Natriumchlorid-Injektionslösung.

4. Die Anwendung der pharmazeutischen Lösung gemäß der Ansprüche 1, 2 oder 3 wird bei der Herstellung von Medikamenten verwendet, mit denen Blutungen aufgrund von Gerinnungsstörungen behandelt werden.

## Revendications

1. Une composition pharmaceutique utile dans le traitement des troubles hémorragiques liés à la coagulation, la composition contenant de la L-ornithine (0,5∼8 g), de l'acide aspartique (1-5 g), de l'arginine (3∼10 g) et de la vitamine B6 (3-10 g par unité).

2. La composition pharmaceutique selon la revendication 1 contenant également l'une ou plusieurs des substances suivantes par unité : isoleucine, leucine, lysine, méthionine, phénylalanine, thréonine, tryptophane, valine, histidine, glycine, alanine, proline, asparagine, cystéine, acide glutamique, sérine, tyrosine, vitamine B1, vitamine B2, vitamine B3, acide pantothénique, biotine, acide folique, vitamine B 12 et vitamine C ; composition pharmaceutique dans laquelle
les dosages respectifs des acides aminés sont les suivants : isoleucine 3∼10 g, leucine 5∼15 g, lysine 3∼10 g, méthionine 0,5~3 g, phénylalanine 0,5~3 g, thréonine 3∼10 g, tryptophane 0.5~3 g, valine 5∼15 g, histidine 3-8 g, glycine 3-8 g, alanine 3∼10 g, proline 3-8 g, asparagine 0,1∼3 g, cystéine 0.1∼3g, acide glutamique 3∼10 g, serine 0,5~5 g, tyrosine 0,1∼3 g ; les dosages respectifs des vitamines B sont les suivants : vitamine B1 1∼2 mg, vitamine B2 1-2 mg, vitamine B3 10∼20 mg, acide pantothénique 3-5 mg, biotine 0,1∼0,2 mg, acide folique 0,1∼0,4 mg, vitamine B 12 2-6 µg, vitamine C 1-3 g.

3. La composition pharmaceutique selon la revendication 1 contenant également une quantité appropriée de 5% de chlorure de sodium et de solution pour injection de dextrose ou de 0,9% de solution pour injection de chlorure de sodium.

4. L'application de la composition pharmaceutique selon la revendication 1, 2 ou 3 dans la préparation de médicaments utilisés pour traiter des troubles hémorragiques liés à la coagulation.
